Europäisches Patentamt

European Patent Office

Office européen des brevets

(11) Publication number: 0 398 361
A2

# EUROPEAN PATENT APPLICATION

(21) Application number: 90109472.2

(22) Date of filing: 18.05.90

(51) Int. Cl.5: C12N 9/72, C12N 15/58,
C12N 5/10

The microorganism(s) has (have) been deposited with Institute for Fermentation under numbers 14872 and 14964.

(30) Priority: 18.05.89 JP 126433/89
22.02.90 JP 42020/90

(43) Date of publication of application:
22.11.90 Bulletin 90/47

(84) Designated Contracting States:
BE CH DE ES FR GB IT LI NL SE

(71) Applicant: THE GREEN CROSS CORPORATION
3-3, Imabashi 1-chome Chuo-ku
Osaka-shi Osaka(JP)

(72) Inventor: Tanabe, Toshizumi, c/o The Green
Cross Corporation
Central Research Lab., 2-1180-1,
Shodai-ohtani
Hirakata-shi, Osaka(JP)
Inventor: Amatsuji, Yasuo, c/o The Green
Cross Corporation
Central Research Lab., 2-1180-1,
Shodai-ohtani
Hirakata-shi, Osaka(JP)
Inventor: Kasai, Shunji, c/o The Green Cross
Corporation
Central Research Lab., 2-1180-1,

Shodai-ohtani
Hirakata-shi, Osaka(JP)
Inventor: Hirose, Masaaki, c/o The Green
Cross Corporation
Central Research Lab., 2-1180-1,
Shodai-ohtani
Hirakata-shi, Osaka(JP)
Inventor: Morita, Masanori, c/o The Green
Cross Corporation
Central Research Lab., 2-1180-1,
Shodai-ohtani
Hirakata-shi, Osaka(JP)
Inventor: Kawabe, Haruhide, c/o The Green
Cross Corporation
Central Research Lab., 2-1180-1,
Shodai-ohtani
Hirakata-shi, Osaka(JP)
Inventor: Arimura, Hirofumi, c/o The Green
Cross Corporatio
Central Research Lab., 2-1180-1,
Shodai-ohtani
Hirakata-shi, Osaka(JP)

(74) Representative: Hansen, Bernd, Dr.
Dipl.-Chem. et al
Hoffmann, Eitle & Partner Patent- und
Rechtsanwälte Arabellastrasse 4
D-8000 München 81(DE)

(54) Human prourokinase variants, methods of producing same, DNA sequences, plasmids and hosts therefor.

(57) A human prourokinase variant deficient in all or at least part of the first loop region or all or at least part of the third region of the epidermal growth factor (EGF) domain of human prourokinase, which shows a long blood half-life comparable to that of the whole EGF domain-deficient PUK variant.

# HUMAN PROUROKINASE VARIANTS, METHODS OF PRODUCING SAME, DNA SEQUENCES, PLASMIDS AND HOSTS THEREFOR

## FIELD OF THE INVENTION

This invention relates to human prourokinase (hereinafter referred to as "human PUK") variants derived from human PUK by modifying its molecular structure, a method of producing the same, DNA sequences coding for said human PUK variants, plasmids with said DNA sequences inserted therein, and transformants harboring said plasmids. More specifically, the invention relates to a series of techniques for providing human PUK variants which comprise deleting, on the gene level, a part of a specific gene region and causing the so-modified gene to be expressed through application of the recombinant DNA technology.

## BACKGROUND OF THE INVENTION

There are two known species of plasminogen activating factor (hereinafter referred to as "PA") involved in fibrinolysis, urokinase (UK) and tissue plasminogen activator (t-PA). UK is a fibrinolytic enzyme, which, thus far, has been purified from human urine and from the culture of human kidney cells, among others. Recently, UK has also become possible to produce UK by utilizing recombinant DNA technology (JP-A-60-180591; the term "JP-A" as used herein means "an unexamined published Japanese patent application" or EP-A-154272).

A serious side effect of UK is that when used in large amounts, it appears to induce degradation and activation of various factors involved in coagulation and fibrinolysis, thus creating a tendency toward spontaneous bleeding. The present inventors found that an inactive precursor to human UK, prourokinase (hereinafter PUK) (JP-A-60-62981 or EP-A-139447; J. Biol. Chem., 260, 12377 (1985)), unlike UK, will dissolve thrombi without creating a tendency toward spontaneous bleeding (Cell Struc. Func., 10, 151 (1985)).

Human PUK is composed of three functional domains, namely the epidermal growth factor (hereinafter abbreviated as "EGF") domain, the kringle domain and the enzyme activity domain (Hoppe-Seyler's Z. Physiol. Chem., 363, 1155 (1982)).

For the three reasons mentioned below, it is presumed that a PUK-specific receptor is present on the liver cell and has an influence on the blood half-life. The three reasons are: firstly, PUK intravenously administered to rats is metabolized mainly in the liver (Suyama et al.: "Fibrinolysis: Current Prospects", Jphn Libbey & Company Ltd., London, pp. 35-43 (1988)); secondly, elimination of the EGF domain from t-PA, which is similar in structure to PUK, results in an increase in blood half-life (Bowne et al.: J. Biol. Chem., 263, 1599 (1988)); and, thirdly, a UK-specific receptor is present on the human monocytic cell line U937 and the EGF domain of UK is involved in UK-receptor binding (Appella et al.: J. Biol. Chem., 262, 4437 (1987)).

The present inventors previously found that human PUK without the EGF domain shows a half-life about three times longer than PUK with the EGF domain in a blood half-life test using rats (EP-A-253241). Deletion of any fairly large segment from a naturally occurring protein, however, may cause a great change in the structure of the original protein thus resulting in various unfavorable changes in properties such as the occurrence of new antigenicity.

## SUMMARY OF THE INVENTION

Accordingly, the following are objectives of the present invention: 1) A human PUK variant showing an increased blood half-life comparable to that of the whole EGF domain-deficient human PUK variant while retaining substantially the same properties as those of human PUK. 2) A human PUK variant having potent thrombolytic activity and very little tendency to cause spontaneous bleeding. 3) A method of producing such human PUK variant. 4) A DNA sequence coding for said variant. 5) A plasmid with said DNA sequence inserted therein. 6) A transformant harboring said plasmid.

These objectives have been attained by a human prourokinase variant comprising human prourokinase deficient in all or at least part of the first loop region of the epidermal growth factor domain and human prourokinase variant deficient in all or at least part of the third loop region of the epidermal growth factor domain, a method of producing said human prourokinase variant comprising the steps of: inserting a DNA sequence coding for said human prourokinase variant into a plasmid; transforming a host with said transformed plasmid; and expressing said DNA sequence, a DNA sequence coding for said human prourokinase variant, a plasmid containing a DNA coding for said human prourokinase variant, and a host transformed with said plasmid.

## BRIEF DESCRIPTION OF THE DRAWINGS

Fig. 1(1)-(2) shows the amino acid sequence and the base sequence of human PUK cDNA.

Fig. 2(1)-(3) shows the processes for the construction of the plasmids pUH7.

Fig. 3 show the processes for the construction of the plasmid pMR304 and pMR305.

Fig. 4 shows the restriction enzyme map of plasmid pSV-$G_1$-NEO.

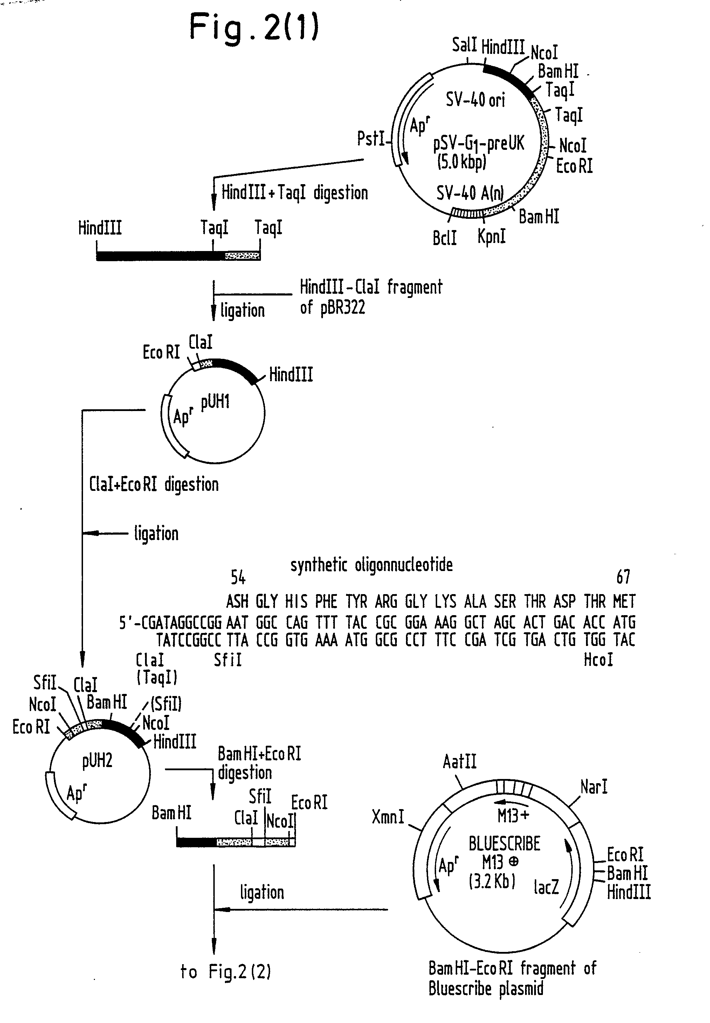

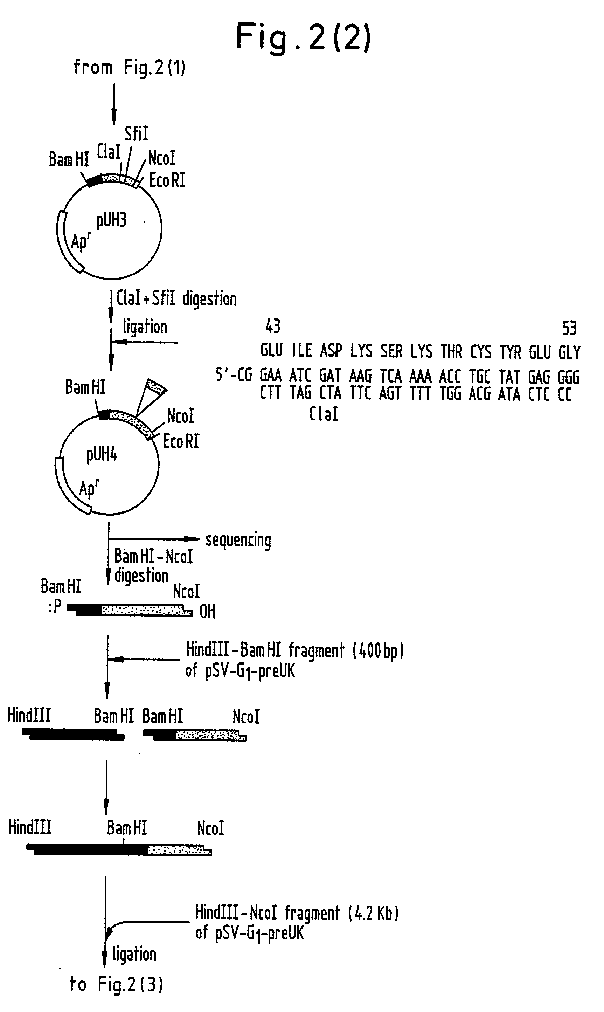

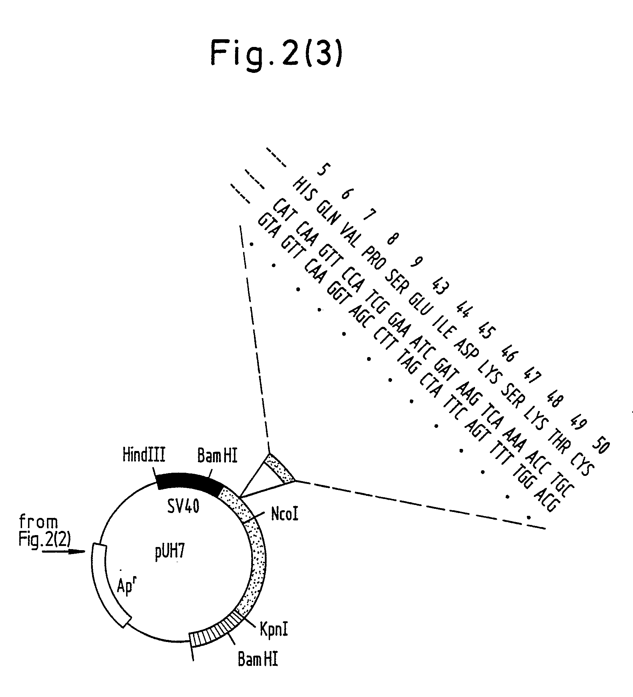

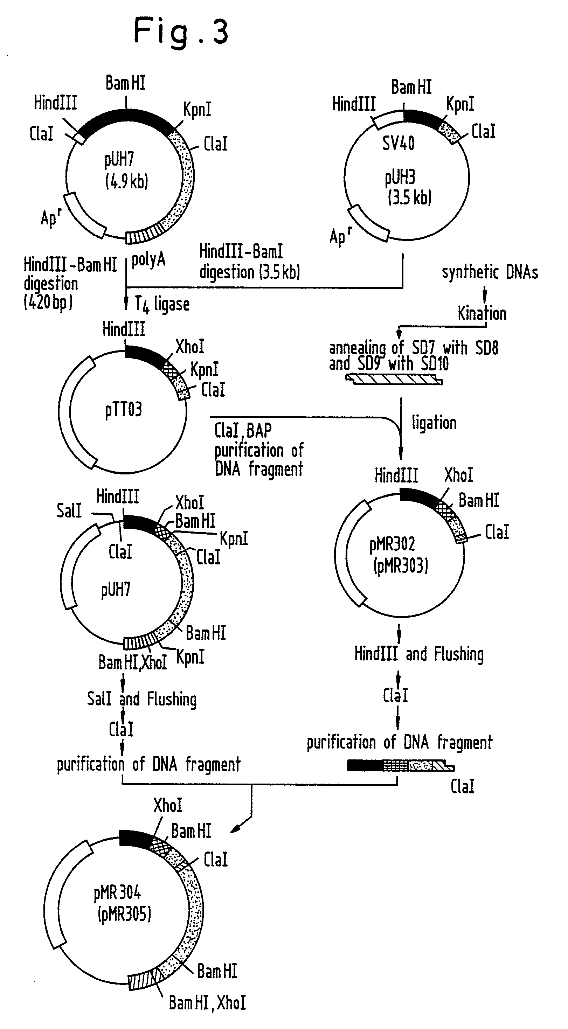

Through Fig. 2 to Fig. 4, ▬ stands for SV40 early promoter-enhancer, ▦ for PUK cDNA, ▦ for splicing junction, ▥ for poly A signal and ▨ for synthetic DNA.

## DETAILED DESCRIPTION OF THE INVENTION

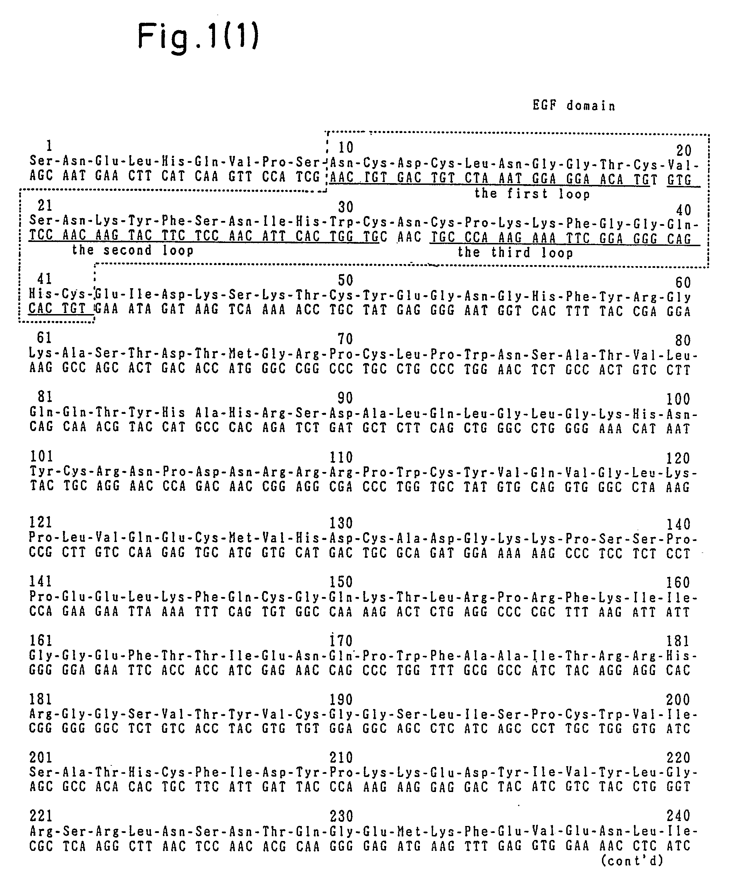

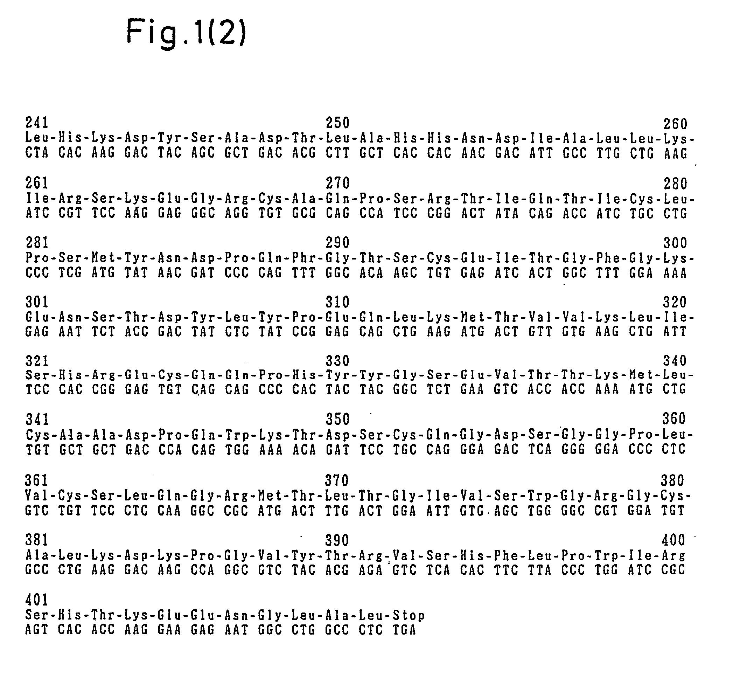

Among the 411 amino acid residues in human PUK (cf. Fig. 1), 49 amino acid residues from the N-terminal serine to the 49th amino acid residue threonine of the mature protein constitute the EGF domain (Riccio, A. et al.: Nucleic Acids Res., 13, 2759-2771 (1985)). The present invention provides a series of techniques for producing a human PUK variant deficient in a part of this EGF domain region.

The EGF domain (Asn-10 to Cys-42) of PUK has a structure consisting of three loops; Asn-10 to Cys-19 form the first loop, Val-20 to Cys-31 form the second, and Cys-33 to Cys-42 form the third.

The human PUK variant according to the present invention is deficient in at least all or part of the first loop region or all or part of the third region in the presence or absence of all or part of the second loop region.

Any part of the EGF domain can be deleted by DNA recombinant technique.

There are teachings suggesting that, in an EGF-EGF receptor system and a UK-UK receptor system, the second loop contributes to the specificity of the binding to the receptor and the first loop stabilizes the bound receptor. In view of the above, a PUK variant deficient in the first and second loops (from Asn-10 to Cys-31) of the EGF domain or a PUK variant deficient in the third loop (from Cys-33 to Cys-42) of the EGF domain and having distortion at the first or second loop region is particularly preferred.

For the deletion of the specific EGF domain region, relevant techniques belonging to the technology of protein engineering can be utilized, for example site-directed deletion (Nucl. Acids Res., 11, 1645 (1983)), site-specific mutagenesis, and use of restriction enzymes and synthetic genes.

PUK cDNA to be used as a starting material can be prepared by the method described in EP-A-154272 or EP-A-253241. An example of the PUK cDNA-containing plasmid usable in the present invention is pSV-$G_1$-preUK (EP-A-154272).

The PUK variant gene obtained by such deletion treatment is inserted into an expression vector system to construct a host-vector system for expression. The host-vector system comprises a host and, in combination therewith, a plasmid vector containing a replicon and a regulatory sequence each derived from a species compatible with the host cells.

Usable as the host are the Escherichia coli strains HB101, C600 and W3110, and Saccharomyces cerivisiae strains GRF18 (JP-A-59-48082 or EP-A-100561) and AH22 (ATCC 38626), among others.

Vectors usable in the present invention contains an origin for initiation of replication and a marker sequence enabling phenotypic selection of desired transformant cells. For instance, it is well known to transform Escherichia coli using the plasmid pBR322 derived from an Escherichia coli (Bolivar et al.: Gene, 2, 95 (1977)). pBR322 has the genes for ampicillin resistance and tetracycline resistance, making it possible to detect transformed cells in a simple and easy manner.

The pBR322 plasmid and other microorganism-derived plasmids have either a promoter utilizable by microorganisms and capable of causing a protein gene under the control thereof to be expressed or a promoter sequence inserted therein. Promoters useful in the described invention are the beta-lactamase (penicillinase) or lactose promoter system (Chang et al.: Nature, 275, 615 (1978); Itakura et al.: Science, 198, 1056 (1977); Goeddel et al.: Nature, 281, 544 (1979)) and the tryptophan (trp) promoter system (Goeddel et al.: Nacl Acids Res., 8, 4057 (1979); EP-A-0036776). Any other microorganism-derived promoters are also useful. Their base sequences have been published in detail and it is possible to insert them into plasmid vectors so that they can perform their function (Siebenlist et al.: Cell, 20, 269 (1980)). Suitable examples of the promoter for use in yeast vectors, for example, are the 3-phosphoglycerate kinase (PGK) promoter (Hitzeman et al.: J. Biol. Chem., 255, 2073 (1968)) and the promoters for other glycolytic system enzymes (Hess, et al.: J. Adv. Enzyme Reg.: 7, 149 (1968); Holland et al.: Biochemistry, 17, 4900 (1978)), such as enolase, glyceraldehyde-3-phosphate dehydrogenase (GAP-DH), hexokinase, pyruvate decarboxylase, phosphofructokinase, glucose-6-phosphate isomerase, 3-phosphoglycerol phosphate mutase, pyruvate kinase, triose phosphate isomerase, phosphoglucose isomerase and glucokinase. Among them, a miniaturized GAP-DH promoter (JP-A-62-175180 or EP-A-218209) and the PGK promoter (Nucleic Acid Res., 10(8), 2625 (1982)) are preferred.

In constructing adequate expression vectors, the transcription termination sequence (terminator) occurring in these genes is inserted into the vectors on the 3′ side of the gene so that poly(A) is added to the mRNA to terminate transcription. Also usable are those promoters in which transcriptional control is possible based on the growth conditions, for example the promoters for alcohol dehydrogenase 2, isocytochrome c, acid phosphatase, enzymes involved in nitrogen metabolism, the above-mentioned glyceraldehyde-3-phosphate dehydrogenase, and enzymes involved in the utilization of maltose or galactose. Any plasmid vector containing a compatible yeast promoter, a replication origin and a terminator sequence can be used.

Additionally useful with the present invention are plasmids having the replication origin of the plasmid pUB110 and the promoter, signal peptide gene and terminator of the α-amylase gene for use in Bacillus natto or Bacillus subtilis, for instance.

Cultured vertebrate cells are also useful (Tissue Culture, Academic Press, Kruse and Patterson, Editors (1973)). Examples of cell lines useful as hosts are VERO, HeLa cells, Chinese hamster ovary (CHO) cell lines, W138, BHK, COS-7, MDCK cell lines, C127, HKG, human kidney cell lines, etc. Expression vectors for use in these cells contain a replication origin, a promoter located upstream from the human PUK variant gene to be expressed, a ribosome binding site, an RNA splicing site, a poly(A) addition site and a terminator sequence.

Where mammalian cells are used, the regulatory function of the expression vector are mostly of the viral origin. For example, the polyoma virus or adenovirus 2 promoter is a promoter in general use and the simian virus 40 (SV40) promoters are used most frequently. The SV40 virus-derived early and late promoters are of particular use since they can be readily obtained from the virus as a fragment containing the replication origin of SV40 [Fiers et al.: Nature, 273, 113 (1978)]. A fragment of the virus which is about 250 bp in size and covers the segment from the HindIII site to the BgII site in the replication origin can be used as well. Any promoter or regulatory sequence (enhancer) associated with the human PUK variant gene to be expressed can be used provided that it is compatible with the host.

Promoter-enhancers to be used in expression vectors for use in animal cells with the present invention are the SV40 early or late gene promoter-enhancer, adenovirus major late promoter region, globulin enhancer-promoter region, RNA virus LTR, metallothionein promoter region, β-actin promoter, etc. The replication origin may be one derived from SV40 or some other virus (e.g. polyoma virus, adenovirus, VSV, BPV) as inserted into the vector, or the replication mechanism of a host chromosome as such. If the vector can be incorporated into a host cell chromosome, the latter suffices. Furthermore, it is possible to use gene amplification systems utilizing the DHFR gene as other high-production systems. Usable signal peptide in the present invention is one derived from the PUK gene, for example, derived from pSV-G₁ preUK (EP-154272). It is noted that the scope of the invention is by no means limited to those host cells, vectors and expression systems mentioned above as typical examples.

In a preferred embodiment of the invention, expression vectors for use in animal cells were constructed by insertion of a gene coding for a human PUK variant at a site downstream from the SV40 early promoter region.

First, the human PUK expression vector pSV-G1-preUK (EP-A-154272) was digested with HindIII and TaqI and a partial human PUK gene fragment containing the coding region for the N terminus to the 10th amino acid Asn was isolated and inserted into the plasmid pBR322 to give a plasmid, pUK1. Then, a synthetic gene coding for the region Asn-54 to Met-67 of human PUK and ending in a ClaI and an EcoRI site was prepared and inserted into the plasmid pUH1 to give a plasmid, pUH2. This synthetic gene contained a site recognizable by a peculiar restriction enzyme, SfiI.

The BaMHI-EcoRI fragment containing only the SfiI site on the synthetic gene was inserted into the Bluescribe plasmid (Vector Cloning Systems) to give a plasmid, pUH3. A synthetic gene coding for Glu-43

4

to Gly-53 and ending in a ClaI and an SfiI site was inserted into said pUH3 at the SfiI-ClaI site thereof to give a plasmid, pUH4, containing a partial human PUK (N terminus to Met-67) gene, without the EGF domain region. Then, the BamHI-NcoI fragment of pUH4 was first ligated to the HindIII-BamHI fragment of pSV-G1-preUK. The resulting fragment was then inserted into pSV-G1-preUK at the HindIII-NcoI site thereof. Thus were constructed expression vector pUH7 without the EGF domain.

Then, 420 bp HindIII-BamHI DNA fragment of pUH7 and 3.5 kb HindIII-BamHI DNA fragment of pUH3 were ligated to each other to thereby obtain plasmid pTT03. This plasmid pTT03 contains SV40 enhancer-promoter region, the PUK signal sequence and a DNA coding for the N-terminal four amino acid of PUK.

Separately, two synthetic DNAs coding for Cys-11 to Asn-32 and Cys-33 to Cys-42 were each prepared. These synthetic DNAs were each ligated to 3.9 kb DNA fragment of pTT03 previously digested with ClaI to obtain plasmids pMR302 and pMR303. Then, 780 bp HindIII-ClaI DNA fragment of pMR302 and 820 bp HindIII-ClaI DNA fragment of pMR303 were each ligated to 4.0 kb DNA fragment of pUH7 previously digested with SalI and ClaI. Thus, PUK variant expression vectors, pMR304 and pMR305 were obtained.

They were introduced into CHO K1 cells for transformation. In this experimental system, clones (high production strains) producing PUK variants in amounts of 40.8 and 88.6 IU/ml/3 days, respectively, were obtained.

The human PUK variants according to the invention can be purified by the known method of purifying human PUK (JP-A-60-62981). In a specific embodiment of the invention, purification was effected by combined column chromatography using chelating Sepharose 6B, anti-UK IgG-Sepharose 4B and p-aminobenzamidine-Sepharose 6B. Chelating Sepharose 6B is particularly effective in rough purification, anti-UK IgG-Sepharose 4B in high-level purification, and p-aminobenzamidine-Sepharose 6B in removing contaminant active-form urokinase (UK).

The thus-obtained PUK variants have a molecular weight ranging from 48,000 to 49,000. That is, a molecular weight of the PUK variant derived from pMR304 (deficient in the first and second loops) is 48,000 and that of the PUK variant derived from pMR305 (deficient in the third loop) is 49,000.

Amino acid sequences of the pMR304-derived PUK variant and the pMR305-derived PUK variant are those deficient in the Cys-11 to Asn-32 region and the Cys-33 to Cys-42 region, respectively, of human PUK as shown in Fig. 1. These PUK variants have DNA base sequences deficient in the base sequence corresponding to the Cys-11 to Asn-32 region and the Cys-33 to Cys-42 region, respectively, of human PUK as shown in Fig. 1.

Analysis of the products thus obtained revealed that the variants are quite comparable in PUK activity to the nonvariant form and that the human PUK variants each is a single-chain proenzyme and entirely convertible to the active form upon plasmin treatment. Furthermore, comparison in blood half-life between the human PUK variants and human kidney cell-derived PUK (J. Biol. Chem., 260, 12377 (1985)) showed that the human PUK variants have a significantly prolonged blood half-life.

The present invention as described herein makes it possible to provide human PUK variants of physiological significance as PA precursors which show a blood half-life significantly longer than human PUK and urokinase and comparable to that of the human PUK variant deficient in the whole EGF region. Because the extent of deletion is small in the human PUK variants according to the invention retain their original structure without substantial changes and the changes in their properties, in particular new antigenicity development, are minimal.

Accordingly, the present invention provides more ideal fibrinolytic enzymes and is expected to be of great value in the field of therapeutics.

In particular, as a result of the practice of the invention, it was suggested that the first and second loops of the EGF domain interact with receptors on liver cells and that deletion of said loops influences (increases) the blood half-life (uptake mainly by liver cells). It was further suggested that when the third loop is maintained, the adjacent kringle structure will not be distorted and, as a result, the affinity for fibrin is maintained.

The following are non-limiting examples of the production of human PUK variants deficient in the first and second loops or in the third loop of the EGF domain through expression in animal cells.

In this example, synthetic DNAs were purified on modified polyacrylamide gels. The restriction enzymes, T4 DNA polymerase, T4 polynucleotide kinase, bacteria-derived alkaline phosphatase (BAP), enzyme kits (such as M13 sequencing kit and ligation kit), and Escherichia coli HB101 and JM109 competent cells used were obtained from Takara Shuzo. The calf intestine alkaline phosphatase used was a product of Boehringer Mannheim. Recombinant DNA techniques were applied as described in "Molecular Cloning", Cold Spring Harbor, New York, Cold Spring Harbor Laboratory (1982).

EXAMPLE

(a) Construction of plasmids

Construction of plasmid pUH1

The EGF domain-coding region (Asn-10-Cys-42) was eliminated from the human PUK gene contained in the human PUK expression vector plasmid pSV-G1-preUK (JP-A-60-180591 and EP-A-154272). pSV-G₁ preUK has a number of TaqI sites and it is difficult to cleave the TaqI site in the vicinity of the EGF domain region exclusively by one restriction enzyme treatment. Therefore, a DNA fragment containing the latter TaqI site was excised. pSV-G1-preUK was digested with the restriction enzymes HindIII and BglII and the resulting DNA fragments were subjected to agarose gel electrophoresis. The gel portion containing an EGF domain-coding region-containing 1.1 kb DNA fragment was excised from the gel and the DNA was recovered. This fragment was then partially digested with TaqI and the digest was subjected to polyacrylamide gel electrophoresis. The presence of the desired 760 bp band was confirmed and this DNA fragment was recovered.

Separately, the plasmid pBR322 was digested with HindIII and ClaI and a 4.3 kb DNA fragment was isolated. This DNA and the previously prepared 760bp HindIII-TaqI DNA fragment were ligated together. This ligation resulted in conversion of the TaqI site at the 3′ end of the 760 bp fragment to a ClaI site. The resulting ligation mixture was used to transform Escherichia coli HB101. The DNA was prepared from each of 24 transformants thus obtained was digested with ClaI and NcoI. The desired plasmid pUH1 (Fig. 2) must be cleaved at one site only with each of ClaI and NcoI. Electrophoresis detected the desired plasmid in 6 clones.

Construction of plasmid pUH2

This plasmid pUH1 was digested with EcoRI and ClaI, the digest was subjected to agarose gel electrophoresis and a DNA fragment about 5.0 kb in size was recovered from the gel. Separately, the following two oligonucleotides were synthesized.

```
              54
              Asn Gly His Phe Tyr Arg Gly Lys Ala Ser
  5'-CGATAGGCCGG  AAT GGC CAC TTT TAC CGC GGA AAG GCT AGC
      TATCCGGCC   TTA CCG GTG AAA ATG GCG CCT TTC CGA TGA
  ClaI (TaqI)  SfiI

           67
  Thr Asp Thr Met

  ACT GAC ACC ATG    56mer
  TGA CTG TGG TAC    54mer
        EcoRI
```

These synthetic oligonucleotides contained the region of Asn-54 to Met-67 of human PUK and ending in ClaI and EcoRI site. The annealing product of two synthetic oligonucleotides was ligated to the above 5.0 kb DNA fragment. The ligation mixture was used to transform Escherichia coli HB101. The DNA prepared from each of 20 transformant strains thus obtained was digested with NcoI, EcoRI and HindIII. The desired plasmid pUH2 must give a DNA fragment about 700 bp in size upon digestion with NcoI and a DNA fragment about 810 bp in size upon digestion with EcoRI and HindIII. As a result of electrophoresis, the desired plasmid was found in 4 clones.

## Construction of plasmid pUH3

Since, in pUH2, there are two SfiI sites in close proximity to each other, a BamHI-EcoRI fragment containing the synthetic gene-derived SfiI site was transferred to another vector. First, pUH2 was digested with BamHI and EcoRI and the digest was subjected to polyacrylamide gel electrophoresis. A 330 bp DNA fragment band was excised from the gel and the DNA was recovered by electroelution.

Separately, the plasmid Bluescribe (Vector Cloning Systems) was digested with BamHI and EcoRI and the thus-obtained DNA fragment about 3.1 kb in size was ligated to the previously prepared 330 bp BamHI-EcoRI fragment. The ligation mixture was used to transform Escherichia coli JM105. The plasmid DNA was prepared from each of 12 selected transformant strains was digested with SfiI and ClaI. The desired plasmid pUH3 must be cleaved only with one site with SfiI and with ClaI. As a result of electrophoresis, 8 clones were found to contain the desired plasmid.

## Construction of plasmid pUH4

pUH3 was digested with ClaI and SfiI, the digest was subjected to agarose gel electrophoresis, and a DNA fragment about 3.1 kb in size was recovered by electroelution. Separately, the following two oligonucleotides were synthesized.

```
        43                                              53
        Glu Ile Asp Lys Ser Lys Thr Cys Tyr Glu Gly

5'-CG GAA ATC GAT AAG TCA AAA ACC TGC TAT GAG GGG
      CTT TAG CTA TTC AGT TTT TGG ACG ATA CTC CC
```

These oligonucleotides contained the region of Glu-43 to Gly-53 of human PUK. The annealing product of two synthetic oligonucleotides was ligated to the above 3.1 kb DNA fragment. The ligation mixture was used to transform Escherichia coli JM105. The plasmid DNA was prepared from each of 10 strains selected for the synthetic gene from among the transformants obtained and digested with BamHI and EcoRI. The desired plasmid pUH4 must give a 360 bp fragment upon the restriction enzyme digestion. As a result of acrylamide gel electrophoresis, two clones harboring pUH4 were obtained.

The plasmids obtained in the above manner were used for the determination of the EGF domain region DNA base sequences. It was found that the respective contemplated deletion regions had been deleted, without any change in the remaining amino acid sequence regions.

## Construction of plasmid pUH7

Then, a partial gene covering the SV40 early gene promoter region to EGF domain-deficient human PUK variant gene was excised from the plasmid pUH4. pUH4 was cleaved with NcoI and then treated with calf intestine alkaline phosphatase for dephosphorylation at the 5′ ends. The DNA was then digested with BamHI and the digest was subjected to polyacrylamide gel electrophoresis. A 360 bp DNA fragment-containing gel portion was excised and the DNA was recovered by electroelution. This DNA was further ligated to a separately prepared 400 bp BamHI-HindIII fragment of the plasmid pSV-G1-preUK. After ligation, the ligation product DNA was recovered by precipitation with ethanol.

Separately, pSV-G1-preUK was digested with HindIII and NcoI and the digest was subjected to electrophoresis. A portion containing a DNA fragment about 4.2 kb in size was excised from the gel and the DNA was recovered by electroelution. This 4.2 kb HindIII-NcoI DNA fragment was ligated to the above-mentioned ligation product. The resultant ligation mixture was used to transform Escherichia coli HB101. The plasmid DNA was extracted from each of 16 strains selected from among the transformant strains obtained and each DNA was digested with HindIII and with HindIII and BglII. The desired plasmid pUH7 must give a DNA fragment about 4.9 kb in size upon HindIII digestion and a fragment about 1.0 kb in size upon HindIII-BglII digestion. As a result of electrophoresis, 3 clones harboring the desired pUH7 were obtained. One of them was selected and this plasmid was prepared in large quantities.

Construction of pTTO3

A plasmid, pTT03, containing the SV40 enhancer-promoter region, the PUK signal sequence and a gene coding for the N-terminal four amino acids of PUK was constructed. For obtaining 5 regions of the SV40 enhancer-promoter with a BamHI recognition site, pUH7 was digested with HindIII and BamHI and a 420 bp fragment was isolated and purified by agarose gel electrophoresis. Separately, pUH3 was digested also with HindIII and BamHI and linearized 3.5 kb DNA fragment was isolated and purified by agarose gel electrophoresis. This fragment was ligated to the above-mentioned 420 bp fragment with DNA ligase. A plasmid giving a 3.5 kb fragment and a 420 bp fragment upon digestion with HindIII and BamHI was named pTT03. Twelve transformants strains were examined and found all to be transformants harboring pTT03 [cf. Fig. 1-(2) ].

Synthesis of oligonucleotides

Four single-stranded DNAs (SD7, SD8, SD9 and SD10) were prepared using an automatic DNA synthesizer (Applied Biosystems model 381A). Their base sequences are shown below. The numbering of an amino acid residue corresponds to that of human PUK (Fig. 1).

```
         9   10  33  34  35                          40
         Ser Asn Cys Pro Lys Lys Phe Gly Gly Gln His Cys

  SD7  5'-CG AAC TGC CCA AAG AAA TTC GGA GGG CAG CAC TGT
  SD8     3'-TTG ACG GGT TTC TTT AAG CCT CCC GTC GTG ACA

            45
         Glu Ile Asp

         GAA AT-3'  (40 bases)
         CTT TAGC-5' (40 bases)


         9   10  11              15                      20
         Ser Asn Cys Asp Cys Leu Asn Gly Gly Thr Cys Val

  SD9  5'-CG AAC TGT GAC TGT CTA AAT GGA GGT ACC TGT GTG·
  SD10    3'-TTG ACA CTG ACA GAT TTA CCT CCA TGG ACA CAC

                            25                  30      32
         Ser Asn Lys Tyr Phe Ser Asn Ile His Trp Cys Asn

         TCC AAC AAG TAC TTC TCC AAC ATT CAC TGG TGC AAC
         AGG TTG TTC ATG AAG AGG TTG TAA GTG ACC ACG TTG

         43  44  45
         Glu Ile Asp

         GAA AT-3'   (76 bases)
         CTT TAG C-5'  (76 bases)
```

Each DNA synthesized was eluted from the column with ammonia, dried by heating at 55°C for 20 hours, and dissolved in distilled water. At this point, the yields were as follows: SD7, 1.75 mg; SD8, 1.61 mg; SD9, 2.81 mg; and SD10, 2.61 mg. A 1/5 portion of each DNA was purified on modified polyacrylamide gel. The final yields were as follows: SD7, 10.2 μg; SD8, 9.01 μg; SD9, 7.7 μg; and SD10, 7.0 μg. The two pairs, SD7-SD8 and SD9-SD10, were subjected to kination and then to annealing and the annealing products were used for the plasmid construction mentioned blow.

## Construction of pMR304 and pMR305

An expression vector for a human PUK variant deficient in the first and second loops of the EGF domain was named pMR304 (an Escherichia coli strain harboring the vector has been deposited, since April 28, 1989, with the Institute for Fermentation, Osaka, 17-85, Juso-Honmachi 2-chome, Yodogawaku, Osaka, Japan, under the designation Escherichia coli HB101/pMR304 and under the deposit number IFO 14872). An expression vector for a human PUK variant deficient in the third loop was named pMR305 (an Escherichia coli strain harboring the vector has been deposited since October 25, 1989 with the Institute for Fermentation, Osaka, under the designation Escherichia coli HB101/pMR305 and under the deposit number IFO 14964). Intermediates for constructing these vectors were named pMR302 and pMR303, respectively. The synthetic DNAs SD7 and SD8 were used in constructing pMR302 while SD9 and SD10 were used in constructing pMR303.

pTT03 was digested with ClaI and dephosphorylated with BAP, and the resulting linear DNA (3.9 kb) was purified by the DEAE paper method. This was ligated to the double-stranded DNA resulting from annealing of each pair of synthetic DNAs as obtained above and the ligation mixture was used to transform JM109 competent cells. Digestion with BamHI and ClaI must give a DNA fragment about 300 bp in size in the case of pMR302 or a DNA fragment about 340 bp in size in the case of pMR303. Screening was made using this as an index and 8 transformant clones were obtained for each of pMR302 and pMR303. The base sequences of the inserted DNA portions, namely the synthetic DNA portions, were confirmed by double-stranded DNA sequencing by the dideoxy method. Plasmid preparation from clones identified with respect to the base sequences gave 582 $\mu$g of pMR302 and 522 $\mu$g of pMR303.

pMR302 and pMR303 were each digested with HindIII, then rendered blunt-ended using T4 DNA polymerase, and digested with ClaI. A pMR302-derived 780-bp DNA fragment were purified on a DEAE paper.

pUH7 was digested with SalI and rendered blunt-ended with T4 DNA polymerase. After further digestion with ClaI, a 4.0-kbp DNA fragment was purified by the DEAE paper method. This DNA fragment was ligated to the DNA fragment excised from pMR302 or pMR303 and the ligation mixture was used to transform JM109 competent cells. Each desired plasmid must be cleaved with ClaI at one site only and digestion with both the restriction enzymes ClaI and XhoI must give three DNA fragments 4.9 kb, 1.4 kb and about 360 bp in size in the case of pMR304 or, in the case of pMR305, three DNA fragments 4.9 kb, 1.4 kb and about 390 bp in size. With these as indices, screening was performed and the desired plasmids were prepared. Thus were obtained 384 $\mu$g of pMR304 and 408 $\mu$g of pMR305.

(b) Introduction into CHO cells of expression vector for human PUK variants deficient in part of EGF domain

The two human PUK variant expression vectors pMR304 and pMR305 were introduced into CHO K1 cells.

## Cell culture medium, serum and reagent

The following medium constituents were used for the cell culture: Ham's F12 (Nissui Pharmaceutical); fetal calf serum (Mitsubishi Kasei Corp., hereinafter abbreviated as "FCS"); Hanks' solution (Nissui Pharmaceutical); lilacillin (synthetic penicillin; Takeda Chemical Industries), streptomycin (Meiji Seika); trypsin (Boehringer Mannheim); and G418 (Sigma). Ham's F12 (hereinafter referred to as "F12 medium") containing 100 mg/liter of lilacillin and 10 mg/liter of streptomycin was prepared, filter sterilized, distributed in 1-liter portions, and stored at 4° C.

## Activity assay of human PUK variants

It was assumed that the human PUK activity of a variant in the culture supernatant was correlated with the activity of natural urokinase (UK). Three days after medium exchange, the supernatant was collected and assayed for plasminogen activator (PA) activity by the fibrin agar plate method (Arch. Biochem., 40, 346-351 (1952)) with a standard UK (Green Cross Corp.) as a reference.

9

DNA introduction into cells (by electroporation)

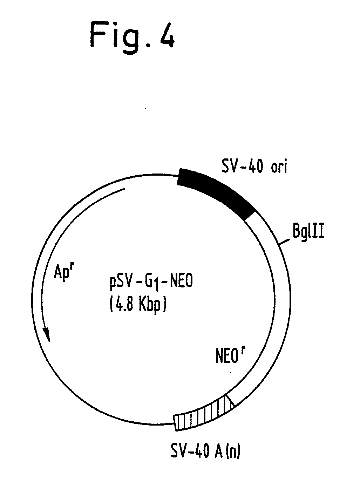

CHO K1 cells in the logarithmic growth phase were dispersed by trypsin treatment, washed twice with Hanks' solution, and suspended in Hanks' solution at a cell density of $10^7$ cells/ml. In a cuvette fitted with electrodes, 0.5 ml of this cell suspension was mixed with 100 ng of pSV-G1-Neo (EP-A-154272; Fig. 4) and 10 μg or 100 μg of the plasmid in question. A 1,000-volt pulse electric field was applied two times and the suspension was allowed to stand for 5 minutes. This cell suspension was diluted with 50 ml of F12 medium supplemented with 10% FCS and the dilution was inoculated in 100-μl portions into a 96-well microplate.

Selection of G418-resistant strains

After electroporation, the dilution was incubated at 37°C for 48 hours and F12 medium containing 10% FCS and 800 μg/ml of G418 was distributed in 100-μl portions into the wells. Thereafter, medium exchange was performed two times a week using F12 medium containing 400 μg/ml of G418 and 10% FCS.

Screening for high productivity strains

After about 2 weeks, colonies at a grown stage were assayed for PA activity and those showing a high activity were cultured on a larger scale. These were inoculated into 10-cm culture dishes at an inoculum size of $1 \times 10^6$ cells per dish. After 3 days, the cells were counted and the PA activity per unit number of cells was calculated.

Introduction of pMR304 and pMR305 into cells and isolation of high expression strains for human PUK variants

CHO K1 cells in the logarithmic growth phase were cotransfected with pMR304 or pMR305 and pSV-G1-Neo by the electroporation method. After about 2 weeks, formation of G418-resistant colonies began. The PA activity values for these colonies as determined on a 96-well microplate scale are shown in Table 1 and Table 2.

Table 1:

| Distribution of CHO K1 clones transfected with pMR304 as grouped in terms of PA activity | | | | | | | |
|---|---|---|---|---|---|---|---|
| | PA activity (IU/ml) | | | | | | |
| | 0 | <5 | <10 | <15 | <20 | >20 | Total |
| After introduction of 10 μg | 7 | 1 | 2 | 0 | 0 | 0 | 10 |
| After introduction of 100 μg | 6 | 1 | 1 | 2 | 0 | 1 | 11 |
| Total | 13 | 2 | 3 | 2 | 0 | 1 | 21 |

Table 2:

| Distribution of CHO K1 clones transfected with pMR305 as grouped in terms of PA activity | | | | | | | |
|---|---|---|---|---|---|---|---|
| | PA activity (IU/ml) | | | | | | |
| | 0 | <5 | <10 | <15 | <20 | >20 | Total |
| After introduction of 10 μg | 21 | 2 | 1 | 0 | 0 | 0 | 24 |
| After introduction 100 μg | 1 | 0 | 1 | 0 | 1 | 0 | 2 |
| Total | 22 | 2 | 1 | 0 | 1 | 0 | 26 |

Among these clones, those that gave the highest PA activity values were cultured on a larger scale in an attempt to isolate high productivity strains. The PA activity data obtained upon larger scale culture in 10-cm culture dishes at an inoculum size of $10^6$ cells per dish as well the data for 96-well plate culture are shown in Table 3.

Table 3:

| PA activities attained upon larger scale culture of strains showing highest activities on 96-well plate | | | | | |
|---|---|---|---|---|---|
| | | PA activity (IU/ml/3days) | | | |
| Plasmids | Clone | 96-well | 10-cm dish | Number of cells in 10-cm dish[a] | PA activity (IU/$10^5$/3 days) |
| pMR304 | 10B4 | 91.22 | 85.57 | $3.08 \times 10^6$ | 28.8 |
| pMR305 | 5B7 | 19.09 | 45.1[40.8] | [$7.24 \times 10^6$] | [5.7] |

Note: a) Number of cells 3 days after inoculation with cells. The values in square brackets were obtained after cell storage in the frozen state, followed by thawing.

c) Purification of human PUK variants

CHO K1 cells transfected with pMR304 (Δ-E$_1$E$_2$-PUK-producing cells) and CHO K1 cells transfected with pMR305 (ΔE$_3$-PUK-producing cells) were used.

The culture supernatants of the above two transfectants were applied to a column of Chelating Sepharose 6B (Pharmacia) carrying zinc ions for adsorption and the column was washed with 20 mM Tris-HCl buffer (pH 7.5) containing 10 KIU/ml aprotinin, and 1M sodium chloride, followed by elution with 20 mM Tris-HCl buffer (pH 7.5) containing 50 mM imidazole, 10 KIU/ml aprotinin and 1M sodium chloride.

The resulting eluate was applied to a column of benzamidine-Sepharose 6B (Pharmacia) equilibrated with 0.1 M phosphate buffer (pH 6.5) containing 0.5 M sodium chloride. Then, the column was washed with 0.1 M phosphate buffer (pH 6.5) containing 10 KIU/ml aprotinin and 0.5 M sodium chloride and the fraction passed through the column was collected.

Sepharose 4B (Pharmacia) to which anti-urine-derived urokinase antibody was bonded was equilibrated with 0.1 M phosphate buffer (pH 6.5) containing 0.5 M sodium chloride. The above-obtained fraction was applied thereto, the column was washed with the same buffer and then, elution was carried out with 0.2 M glycine-HCl buffer (pH 2.5) containing 0.5 M sodium chloride.

The eluate was further applied to a column of benzamidine-Sepharose 6B and the fraction passed through the column was collected.

The thus-obtained fraction was dialyzed against 0.1 M phosphate buffer (pH 6.2).

pMR304-derived human PUK variant (hereinafter ΔE$_1$E$_2$-PUK) and pMR305-derived human PUK variant (hereinafter AE$_3$-PUK) both showed specific activity of 150,000 IU/mg protein when treated with plasmin.

d) Properties of human PUK variants

## Molecular weight

A molecular weight of human PUK variants was determined by SDS-polyacrylamide gel electrophoresis (SDS-PAGE) in accordance with the method of Laemmli (Nature, 227, 680 (1970)).

189-350 IU of $\Delta E_1 E_2$-PUK and $\Delta E_3$-PUK was each boiled at 100°C for 10 minutes in a reducing solution containing 2% 2-mercaptoethanol, 2% SDS, 1% glycine and 50 mM Tris-HCl buffer (pH 6.8). Each reaction mixture was applied onto 10 to 20% gradient gel (Daiichi Kagaku Yakuhin) and electrophoresis was carried out at a constant current of 30 mA for 2 hours. The following molecular weight markers were used: phosphorylase b, 94,000; bovine serum albumin, 67,000; ovalbumin, 43,000; carbonic anhydrase, 30,000; trypsin inhibitor, 20,100; and $\alpha$-lactalbumin, 14,400 (Pharmacia).

After electrophoresis, the gel were stained by Coomassie Brilliant Blue R-250.

As a result, bands of $\Delta E_1 E_2$-PUK and $\Delta E_3$-PUK were found at 48K and 49K, respectively. This results suggested that human PUK variants according to the present invention has a single-stranded molecular structure.

## Enzyme kinetics

Glt-Gly-Arg-MCA (hereinafter abbreviated as MCA), 7-amino-4-methyl-Coumarin (hereinafter abbreviated as AMC) were commercially available from the Peptide Research Institute. The UK standard and plasmin used were products of The Green Cross Corporation.

A 100 $\mu$l portions of a 200 $\mu$l/ml solution of human PUK variant $\Delta E_1 E_2$-PUK and $\Delta E_3$-PUK) were mixed with 100 $\mu$l of a 0.2 cu/ml solution of plasmin. As a buffer solution for PUK variants and plasmin, 0.1 M Tris-HCl buffer (pH 8.4) containing 0.1 M NaCl and 10% gelatin was used. The resulting mixtures were incubated at 37°C for 10 minutes. Then, a 1.0, 0.5, 0.25, 0.125 and 0.625 mM solution of MCA previously maintained at 37°C were each added to the mixtures. After further incubation at 37°C for 3 minutes, 2 ml of a 15% acetic acid solution was added to the mixture to terminate the reaction. For comparison, naturally occurring human PUK (n-PUK; EP-A-139447) was used. Fluorescence intensity of the resulting mixture was measured using a spectrophotofluorometer at an excitation wavelength of 370 nm and a fluorescence wavelength of 460 nm. The concentration of AMC produced by the enzyme reaction was calculated with taking the fluoresence intensity of 10 $\mu$M AMC as 100.

The kinetic constants, Km and Vmax, were calculated in accordance with the Lineweaver-Burk plot method (Segel, I.H. (1976) Biochemical Calculations, 2nd ed. John Wiley & Sons, Inc., New York). kcat was calculated by the following equation on the ground that 1 IU of UK corresponded to $1.33 \times 10^{-7}$ $\mu$mole.

$$kcat = \frac{Vmax}{1.33 \times 10^{-7}} \ (min.^{-1})$$

The kinetic constants of each human PUK variant are shown in Table 4.

Table 4:

| Kinetic constant of human PUK variants | | | |
|---|---|---|---|
| Sample | KM ($\mu$M) | kcat $\times 10^2$ (min.$^{-1}$) | kcat/Km |
| n-PUK | 355 | 84 | 2.4 |
| $\Delta E_1 E_2$-PUK | 340 ± 2.4 | 91 ± 11 | 2.7 ± 0.3 |
| $\Delta E_3$-PUK | 409 ± 12.1 | 101 ± 13 | 2.5 ± 0.2 |
| Note: The value shown in Table 8 is the mean ± standard deviation. The measurement was carried out once in the case of n-PUK and twice in the case of human PUK variants. | | | |

As shown in Table 4, there is no significant difference in kinetic constant between human PUK variants and natural human PUK.

Half-life in Blood

The following samples were tested.
   a) n-PUK (natural): naturally occurring PUK derived from HGK cell (cf. EP-A-139447).
   b) n-PUK (recombinant): PUK produced by CHO K1 cells trasnsformed with pSV-G1-preUK.
   c) $\Delta E_1 E_2 E_3$-PUK: human PUK variant deficient in the entire EGF domain.
   d) $\Delta E_1 E_2$-PUK: human PUK variant deficient in the first loop and the second loop of the EGF domain.
   e) $\Delta E_3$-PUK: human PUK variant deficient in the third loop of the EGF domain.

The above five samples were labeled with $^{125}$I by the lactoperoxidase Enzymobeads (BIO-RAD) method. The radiochemical specific activity of $^{125}$I-PUK thus obtained was calculated from protein content and radioactivity determined by the absorbance at 280 nm. As a result, specific activity of each sample fell within the range from 11000 to 56000 cpm/IU.

The sample solution was diluted with physiological saline so as to give a PUK concentration of $2 \times 10^4$ IU/ml (human albumin concentration: 5%) and was administered to Wister male rats (6-week-old) in a dose of 1 ml/kg through tail vein.

Rats were anesthetized with 35 mg/kg i.m. of ketamine hydrochloride (trade name: Ketalar® produced by Sankyo) and 1.5 g/kg i.m. of urethane (Nakarai Chemical) and fixed on the back. An atom venous catheter (3Fr) filled with a 3.8% solution of sodium citrate (The Green Cross Corporation) was inserted into left carotid artery. 1, 2, 3, 5, 7, 10, 15 and 20 minutes after the administration of samples, blood was collected from each rat using JMS 1 ml-volume of disposal syringe containing 30 $\mu$l of a 3.8 % solution of sodium citrate so that blood was sucked up to the scale of 330 ml (300 ml in terms of blood). The sucked blood was shaken in the syringe and the whole sucked blood was measured for radioactivity using a $\gamma$-counter. After radioactivity of the blood was measured, the whole blood was centrifuged at 3000 rpm for 10 minutes to obtain 100 ml of plasma. The thus-obtained plasma was rapidly frozen and stored at -20° C.

Radioactivity of plasma was measured by a $\gamma$-counter (COMPUGAMMA 1282 model, LKB WALLAC).

Time course of radioactivity of blood was measured and calculated in terms of % of dose. A half-life in blood was calculated by commercially available software.

A half-life in blood (T1/2$\alpha$) is shown in Table 5.

13

Table 5:

| T1/2$\alpha$ (min.) | |
| --- | --- |
| Sample | Radioactivity |
| n-PUK (Natural type) | 2.49 ± 0.17 |
| n-PUK (recombinant) | 2.25 ± 0.54 |
| $\Delta E_1 E_2 E_3$-PUK | 5.65 ± 0.66 |
| $\Delta E_1 E_2$-PUK | 6.33 ± 1.00 |
| $\Delta E_3$-PUK | 5.39 ± 0.86 |
| Note: Each value means the mean ± standard deviation (n = 8). | |

As shown in Table 5, there is a significant difference in half-life in blood such that human PUK variants according to the present invention show a half-life (in terms of radioactivity) about two to three times longer than n-PUKs ($p < 0.05$). $\Delta E_1 E_2$-PUK and $\Delta E_3$-PUK showed T1/2$\alpha$ comparable to that of $\Delta E_1 E_2 E_3$-PUK.

While the invention has been described in detail and with reference to specific embodiments thereof, it will be apparent to one skilled in the art that various changes and modifications can be made therein without departing from the spirit and scope thereof.

## Claims

1. A human prourokinase variant comprising: human prourokinase deficient in all or at least part of the first loop region of the epidermal growth factor domain.

2. A DNA sequence coding for the human prourokinase variant of Claim 1.

3. A plasmid containing a DNA sequence coding for the human prourokinase variant of Claim 1.

4. A host transformed with the plasmid of Claim 3.

5. The host transformed with the plasmid of Claim 3, wherein said host is an animal cell.

6. A human prourokinase variant comprising: human prourokinase deficient in all or at least part of the first loop region and all or at least part or the second loop region of the epidermal growth factor domain.

7. A DNA sequence coding for the human prourokinase variant of Claim 6.

8. A plasmid containing a DNA sequence coding for the human prourokinase variant of Claim 6.

9. A host transformed with the plasmid of Claim 8.

10. The host transformed with the plasmid of Claim 8, wherein said host is an animal cell.

11. A human prourokinase variant comprising: human prourokinase deficient in all or at least part or the third loop region of the epidermal growth factor domain.

12. A DNA sequence coding for the human prourokinase variant of Claim 11.

13. A plasmid containing a DNA sequence coding for the human prourokinase variant of Claim 11.

14. A host transformed with the plasmid of Claim 13.

15. The host transformed with the plasmid of Claim 13, wherein said host is an animal cell.

16. A method of producing a human prourokinase variant comprising the steps of:
inserting a DNA sequence coding for a human prourokinase variant deficient in all or at least part of the first loop region the epidermal growth factor domain into a plasmid;
transforming a host with said plasmid; and
expressing said DNA sequence.

17. The method of producing a human prourokinase variant claimed in Claim 16, where said DNA sequence is inserted downstream from the SV40 early promoter region, and said transformed host is CHO K1 cells.

18. A method of producing a human prourokinase variant comprising the steps of:
inserting a DNA sequence coding for a human prourokinase variant deficient in all or at least part of the third loop region of the epidermal growth factor domain into a plasmid;
transforming a host with said plasmid; and
expressing said DNA sequence.

19. The method of producing a human prourokinase variant claimed in Claim 18, where said DNA sequence is inserted downstream from the SV40 early promoter region, and said transformed host is CHO

K1 cells.

Claims for the following Contracting State: ES

1. A method of producing a human prourokinase variant comprising the steps of:
inserting a DNA sequence coding for a human prourokinase variant deficient in all or at least part of the first loop region the epidermal growth factor domain into a plasmid;
transforming a host with said plasmid; and
expressing said DNA sequence.

2. The method of producing a human prourokinase variant claimed in Claim 1, where said DNA sequence is inserted downstream from the SV40 early promoter region, and said transformed host is CHO K1 cells.

3. A method of producing a human prourokinase variant comprising the steps of:
inserting a DNA sequence coding for a human prourokinase variant deficient in all or at least part of the third loop region of the epidermal growth factor domain into a plasmid;
transforming a host with said plasmid; and
expressing said DNA sequence.

4. The method of producing a human prourokinase variant claimed in Claim 3, where said DNA sequence is inserted downstream from the SV40 early promoter region, and said transformed host is CHO K1 cells.

# Fig.1(1)

EGF domain

```
 1                                        10                              20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG
                                             the first loop
 21                                       30                              40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG
         the second loop                          the third loop
 41                                       50                              60
His-Cys-Glu-Ile-Asp-Lys-Ser-Lys-Thr-Cys-Tyr-Glu-Gly-Asn-Gly-His-Phe-Tyr-Arg-Gly
CAC TGT GAA ATA GAT AAG TCA AAA ACC TGC TAT GAG GGG AAT GGT CAC TTT TAC CGA GGA
 61                                       70                              80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT
 81                                       90                              100
Gln-Gln-Thr-Tyr-His Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT
 101                                      110                             120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG
 121                                      130                             140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT
 141                                      150                             160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT
 161                                      170                             181
Gly-Gly-Glu-Phe-Thr-Thr-Ile-Glu-Asn-Gln-Pro-Trp-Phe-Ala-Ala-Ile-Thr-Arg-Arg-His-
GGG GGA GAA TTC ACC ACC ATC GAG AAC CAG CCC TGG TTT GCG GCC ATC TAC AGG AGG CAC
 181                                      190                             200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC
 201                                      210                             220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT
 221                                      230                             240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC
                                                               (cont'd)
```

# Fig.1(2)

```
241                               250                                    260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                               270                                    280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                               290                                    300
Pro-Ser-Met-Tyr-Asn-Asp-Pro-Gln-Phr-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA

301                               310                                    320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                               330                                    340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG

341                               350                                    360
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC

361                               370                                    380
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

381                               390                                    400
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC

401
Ser-His-Thr-Lys-Glu-Glu-Asn-Gly-Leu-Ala-Leu-Stop
AGT CAC ACC AAG GAA GAG AAT GGC CTG GCC CTC TGA
```

EP 0 398 361 A2

# Fig.2(1)

to Fig.2(2)

# Fig.2(2)

from Fig.2(1)

SfiI

BamHI  ClaI   NcoI
              EcoRI

pUH3

Apʳ

ClaI + SfiI digestion

ligation

```
                                43                                              53
                                GLU ILE ASP LYS SER LYS THR CYS TYR GLU GLY
                         5'-CG GAA ATC GAT AAG TCA AAA ACC TGC TAT GAG GGG
                             CTT TAG CTA TTC AGT TTT TGG ACG ATA CTC CC
                                   ClaI
```

BamHI

NcoI
EcoRI

pUH4

Apʳ

sequencing

BamHI − NcoI
digestion

BamHI
:P ▇▇▇▒▒▒▒▒ OH
              NcoI

HindIII − BamHI fragment (400 bp)
of pSV-G₁-preUK

HindIII      BamHI BamHI        NcoI
▇▇▇▇▇▇▇▇     ▇▇▒▒▒▒▒▒

HindIII      BamHI        NcoI
▇▇▇▇▇▇▇▇▇▇▇▇▇▒▒▒▒▒▒

HindIII − NcoI fragment (4.2 Kb)
of pSV-G₁-preUK

ligation

to Fig.2(3)

# Fig.2(3)

```
                                              5   6   7   8   9   43  44  45  46  47  48  49  50
                              HIS GLN VAL PRO SER GLU ILE ASP LYS SER LYS THR CYS
                              CAT CAA GTT CCA TCG GAA ATC GAT AAG TCA AAA ACC TGC
                              GTA GTT CAA GGT AGC CTT TAG CTA TTC AGT TTT TGG ACG
```

HindIII    BamHI

SV40    NcoI

from Fig.2(2)    pUH7    Ap r

KpnI
BamHI

# Fig.3

# Fig. 4

SV-40 ori

BglII

Ap$^r$

pSV-G$_1$-NEO
(4.8 Kbp)

NEO$^r$

SV-40 A(n)